# EUROPEAN PATENT APPLICATION

(11) **EP 4 640 271 A1**
(43) Date of publication of application: **29.10.2025**
(21) Application number: 25161357.6
(22) Date of filing: 03.03.2025
(51) Int. Cl.: A61N 7/02, A61B 8/00, A61N 7/00, A61B 17/00

(54) **HIGH INTENSIVE FOCUSED ULTRASOUND PROBE**

(30) Priority: 23.04.2024 KR 20240053764
(71) Applicant: VIOL Co. Ltd., Bundang-gu Seongnam-si, Gyeonggi-do, 13510 (KR)
(72) Inventor: LEE, Sangjin, 13510 Seongnam-si (KR); KIM, Jongkil, 13510 Seongnam-si (KR); JEON, Yeonggi, 13510 Seongnam-si (KR)
(74) Representative: Zimmermann, Tankred Klaus

(57) **Abstract**

Disclosed is a high-intensity focused ultrasound (HIFU) probe for noninvasive skin treatment. The probe includes an external rotating shaft driven by a motor, an internal rotating shaft that moves a transducer within a cartridge, and a magnet coupler that magnetically couples the shafts across a first sidewall of the cartridge. This magnetic coupling eliminates the need for sealing components, such as corrugated pipe-structured sealing members, preventing leakage of the ultrasound transmission medium. As a result, the design is simplified, manufacturing costs are reduced, and mass production efficiency is improved. Additionally, by removing sealing constraints, the transducer's movable range is expanded, enabling broader ultrasound treatment coverage in a single procedure.

## Description

### [Technical Field]

Embodiments of the present disclosure relate to an ultrasound probe, and particularly, to a high intensive focused ultrasound probe that noninvasively treats skin by using a high intensive focused ultrasound (HIFU).

### [Background Art]

Skin procedure using a high intensive focused ultrasound (HIFU) has recently been in spotlight. This is a technology of treating skin by using an effect (wrinkle removal, skin elasticity improvement, and the like) occurring when high intensive acoustic energy is focused on a local site in a body by using the high intensive focused ultrasound to increase temperature and thus a degenerated tissue is regenerated due to thermal variations occurring in the local site in the body.

A device that treats skin by using the high intensive focused ultrasound includes a transducer. The transducer generates high intensive ultrasound from an input power source and outputs the high intensive ultrasound. A general high intensive focused ultrasound device uses a circular single-element ultrasound transducer as a transducer. That is, a method is used to transmit strong ultrasound energy to a treatment site through the circular single-element ultrasound transducer.

In order to perform skin treatment or handling on a wider site with one-time procedure, it is necessary to form a plurality of points where ultrasound energy is focused. To this end, single-element ultrasound transducer-type ultrasound treatment devices in the related art adopt a method of mechanically moving the single-element ultrasound transducer so that ultrasound treatment or handling can be performed on a wider range with one-time procedure.

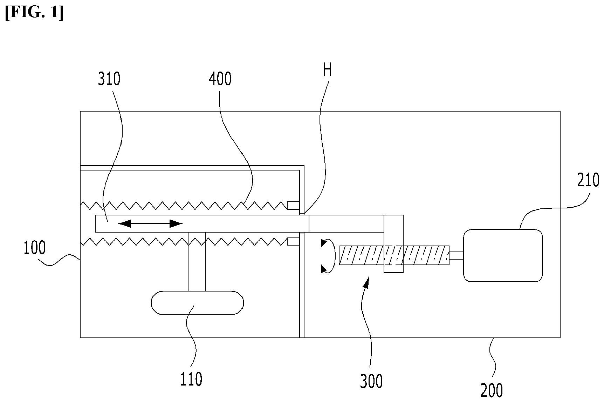

The ultrasound probe of the ultrasound treatment device in which the ultrasound transducer is mechanically moved generally includes a cartridge 100 in which an ultrasound transducer 110 is installed therein and a handpiece 200 as in the example of FIG. 1. The inside of the cartridge 100 is filled with a liquid ultrasound transmission medium, and the handpiece 200 includes a motor 210 for implementing a one-dimensional movement of the ultrasound transducer 110 in a specific direction.

The ultrasound transducer 110 receives power from the motor 210 through a transport mechanism 300 and makes a linear reciprocating motion in a specific direction. The transport mechanism 300 is configured to convert a rotational motion of an output shaft of the motor 210 into a linear motion and transmit the linear motion to the ultrasound transducer 110. In the related art, the transport mechanism 300 that converts the rotational motion into the linear motion and transmits the linear motion to the ultrasound transducer 110 has adopted a lead screw method as in the example of FIG. 1.

As described above, the inside of the cartridge 100 is filled with a liquid substance being an ultrasound transmission medium. Due to the properties of the liquid substance, the liquid substance may be leaked to the outside through even a small gap. Therefore, it is necessary to seal a portion of the cartridge 100 where the ultrasound transmission medium is expected to leak. In particular, in the configuration as illustrated in FIG. 1 in which a transfer shaft 310 penetrates the cartridge 100, it is necessary to seal a portion H (shaft penetration hole) through which the transfer shaft 310 penetrates.

The related art adopts a configuration in which a flexible corrugated pipe-structured sealing member 400 is used to seal the portion through which the transfer shaft 310 penetrates. The sealing member 400 is firmly fixed to one sidewall of the cartridge 100 so as to cover the shaft penetration hole H while wrapping at least a part of the transfer shaft 310 within the cartridge, thereby sealing the penetration hole H while guaranteeing the linear motion characteristics of the transfer shaft 310 within the cartridge 100.

However, such a configuration has the disadvantage of increasing the manufacturing cost due to the use of a separate sealing member and reducing the mass productivity of a product due to structural difficulties in assembly. In particular, when the transfer shaft moves to one side to the extent that the sealing member is completely folded and is not foldable any more, there is a problem that the movement of the transfer shaft is restricted by the completely folded sealing member. That is, there is a structural problem that the movable range of the transducer is restricted by the corrugated pipe-structured sealing member.

Korean Patent Publication No. 10-2012-0140288 (Published on December 31, 2012)

Korean Patent Publication No. 10-2014-0141062 (Published on December 10, 2014)

### [Disclosure]

### [Technical Problem]

An object of the present disclosure is to provide a high intensive focused ultrasound probe that is structurally simple and adopts a power transmission method requiring no separate sealing member for preventing leakage of an ultrasound transmission medium.

Another object of the present disclosure is to provide a high intensive focused ultrasound probe that can increase a movable range of a transducer compared to the configuration of the related art under the premise of the same volume of space and thus can perform ultrasound treatment or handling over a wider range with one-time medical procedure.

Problems to be solved by the present disclosure are not limited to the aforementioned problems, and the other unmentioned problems will be clearly understood by those skilled in the art from the following description.

### [Technical Solution]

The present disclosure is applied to a skin treatment device that noninvasively treats skin by using a high intensive focused ultrasound (HIFU), and provides a high intensive focused ultrasound probe including an external rotating shaft configured to rotate inside the handpiece by a motor, an internal rotating shaft configured to move a transducer while rotating inside the cartridge, and a magnet coupler configured to magnetically couple the external rotating shaft and the internal rotating shaft with a first sidewall of the cartridge interposed between the external rotating shaft and the internal rotating shaft.

The magnet coupler may include a first coupler coupled to the external rotating shaft and making a synchronized rotational motion, and a second coupler coupled to the internal rotating shaft and forming magnetic coupling with the first coupler with the first sidewall interposed between the first coupler and the second coupler.

As an embodiment, one of the first coupler and the second coupler may be a magnetic material and the other one of the first coupler and the second coupler may be a permanent magnet.

As another embodiment, the first coupler and the second coupler may be permanent magnets, and a magnetic pole of a surface of the first coupler and a magnetic pole of a surface of the second coupler may be opposite to each other, the two surfaces being in close contact with each other with the first sidewall interposed therebetween.

As another embodiment, a plurality of coupling magnets may be separately mounted at uniform intervals along a rotation direction on a surface of the first coupler and a surface of the second coupler, the two surfaces facing each other with the first sidewall interposed therebetween. In this case, coupling magnets mounted on the surface of the first coupler may be arranged so that magnetic poles of sides exposed to an outside are alternated with respect to the rotation direction, and coupling magnets mounted on the surface of the second coupler may be arranged so that magnetic poles of sides exposed to the outside are alternated with respect to the rotation direction.

A plurality of balls or needle pins may be installed on the surface of the first coupler and the surface of the second coupler, the two surfaces facing each other with the first sidewall interposed therebetween. In this case, at least a part of the balls or the needle pins may protrude from the surfaces facing each other to support rotational motions of the first coupler and the second coupler in a state of being in contact with the first sidewall.

A ring-shaped internal rotation guide may be further formed on an inner surface of the first sidewall where the magnet coupler is located. In addition, a ring-shaped external rotation guide may be further formed on an outer surface of the first sidewall corresponding to the internal rotation guide. In this case, the magnet coupler may be arranged in an internal coupler receiving portion and an external coupler receiving portion respectively partitioned in an inside and an outside of the first sidewall by the internal rotation guide and the external rotation guide

A first lubricating layer may be formed by a lubricant between the first coupler and a first sidewall of the external coupler receiving portion. In addition, a second lubricating layer may be formed by a lubricant between the second coupler and a first sidewall of the internal coupler receiving portion.

The internal rotating shaft may be configured in the form of a lead screw having threads formed along its peripheral surface. In this case, a movable block having a fastening hole screw-coupled with the threads may be coupled with the internal rotating shaft and the transducer is connected to such a movable block, so that skin treatment or handing on a wider site can be performed due to the movement of the transducer together with the movable block moving on the internal rotating shaft during the rotation of the internal rotating shaft.

An internal space of the cartridge may be divided by a space partition plate arranged therein into a first space and a second space isolated from the first space.

The first space may be filled with a liquid ultrasound transmission medium, and the transducer and the internal rotating shaft may be arranged in the first space filled with the liquid ultrasound transmission medium. In addition, a circuit board that controls the transducer may be arranged in the second space.

At least two detection elements that detect a position of the movable block may be mounted at a distance from each other on the circuit board. In addition, an element to be detected may be arranged on a surface of the movable block adjacent to the circuit board.

Preferably, the detection element may be a Hall element, and the element to be detected may be a permanent magnet.

In the first space, a shaft that guides a one-dimensional linear motion of the movable block may be arranged. In this case, the shaft may be parallel to the internal rotating shaft and formed as one or re more.

### [Advantageous Effects]

In accordance with a high intensive focused ultrasound probe according to embodiments of the present disclosure, a first coupler and a second coupler, which implement coupling between shafts (an internal rotating shaft and an external rotating shaft), are not directly connected, but are indirectly connected through magnetic coupling with a sidewall (first sidewall) interposed therebetween. Accordingly, there is no need to process a hole (hole through which the shaft passes) in a cartridge filled with a liquid ultrasound transmission medium (for example, degassed water).

In addition, since the hole (hole through which the shaft passes) is not required, there is no need to consider a configuration (for example, the corrugated pipe-structured sealing member in the related art) for preventing leakage of the ultrasound transmission medium. That is, by adopting a power transmission method (magnetic coupling) that is structurally simple and requires no separate configuration for preventing the leakage of the ultrasound transmission medium (for example, degassed water) filled in the cartridge, there is an advantageous effect in improving the assembling efficiency and mass productivity of a product.

In addition, since the hole (hole through which the shaft passes) is not required in the cartridge, there is no need to consider sealing during a product design process, which can have the effect of improving the degree of freedom of design, and since an element (sealing member) for restricting a movable range is not required, the movable range of a transducer is increased compared to the configuration in the related art, which has the advantage of allowing ultrasound treatment or handling over a wider range with one-time procedure.

### [Description of Drawings]

FIG. 1 is a schematic view schematically illustrating the configuration of an ultrasound probe according to the related art.
FIG. 2 is a device configuration diagram schematically illustrating the overall configuration of a skin treatment device adopting a high intensive focused ultrasound probe according to embodiments of the present disclosure.
FIG. 3 is a cut perspective view illustrating an internal configuration of the high intensive focused ultrasound probe according to embodiments of the present disclosure.
FIG. 4 is a view illustrating a state in which a cartridge is detached from the high intensive focused ultrasound probe illustrated in FIG. 3.
FIG. 5 is a schematic view schematically illustrating a main configuration of the high intensive focused ultrasound probe according to embodiments of the present disclosure illustrated in FIG. 3.
FIG. 6 is a view illustrating a preferred embodiment of a magnet coupler illustrated in FIG. 5.
FIG. 7 is a view illustrating another preferred embodiment of the magnet coupler illustrated in FIG. 5.
FIG. 8 is a view illustrating further another preferred embodiment of the magnet coupler illustrated in FIG. 5.
FIG. 9 is a view illustrating a preferred modified example of the magnet coupler illustrated in FIG. 5.
FIG. 10 is a view illustrating a preferred modified example of a first sidewall illustrated in FIG. 5.

### [Mode for Invention]

Hereinafter, preferred embodiments of the present disclosure are described in detail with reference to the accompanying drawings.

For reference, in the description of embodiments of the present disclosure, the same or similar components are given the same reference numerals and duplicate descriptions thereof are omitted. Even when it is determined that detailed descriptions of related publicly-known technologies may obscure the subject matter of the embodiments disclosed in the present specification, the detailed descriptions thereof are omitted.

In addition, the suffixes "module" and "unit" for components used in the following description are given or used interchangeably only for the convenience of writing a specification, and do not have distinct meanings or roles in themselves.

In addition, in the description of embodiments of the present disclosure, it should be noted that the accompanying drawings are intended only to help easily understand the embodiments disclosed in the present specification and are not intended to limit the technical spirit disclosed in the present specification, and the present disclosure includes all modifications, equivalents, or substitutes included in the spirit and technical scope of the present disclosure.

In addition, in the description of embodiments of the present disclosure, terms including ordinal numbers such as first and second may be used to describe various components, but the components are not limited by the terms. The terms are used only to distinguish one component from another component.

In addition, when it is described that one component is "connected" or "coupled" to another component, it should be understood that one component may be directly connected or coupled to the another component, but another component may exist between the two components.

On the other hand, when it is described that one component is "directly connected to" or "directly coupled to" another component, it should be understood that another component does not exist between the two components.

In addition, terms such as "includes", "comprises" or "has" used in the description of embodiments of the present disclosure are intended to designate the presence of features, numbers, steps, operations, components, parts, or combinations of the present disclosure, and should be understood as not excluding in advance the possibility of the presence or addition of one or more other features, numbers, steps, operations, components, parts or combinations thereof.

In addition, the fact that a component is "in front," "behind," "above," or "below" another component includes not only the case where it is directly adjacent to the another component and is disposed "in front," "behind," "above," or "below," but also the case where another component is further disposed therebetween unless otherwise specified.

The drawings are intended only to help understand the spirit of the present disclosure, and should not be construed as limiting the scope of the present disclosure. In addition, it should be noted that the relative thickness, length, or size in the drawings may be exaggerated for the convenience and clarity of explanation.

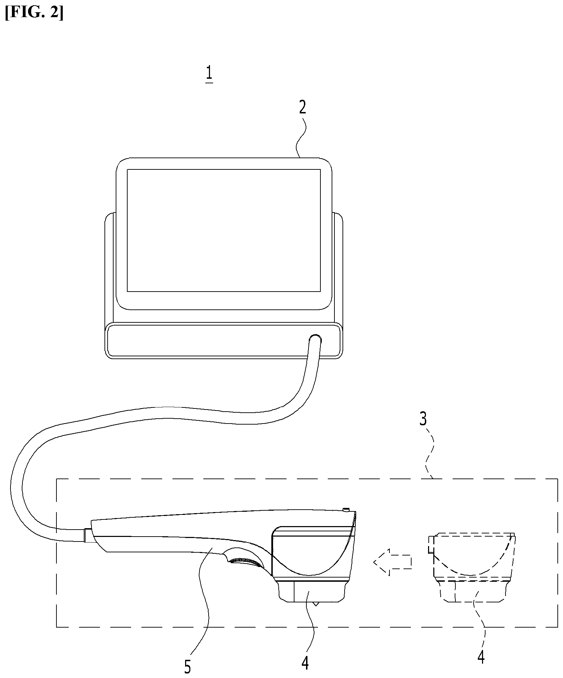

FIG. 2 is a device configuration diagram schematically illustrating the overall configuration of a skin treatment device adopting a high intensive focused ultrasound probe according to embodiments of the present disclosure. First, the configuration of the skin treatment device adopting the high intensive focused ultrasound probe according to embodiments of the present disclosure is briefly described with reference to FIG. 2.

The skin treatment device related to the present disclosure is a device that noninvasively treats or handles skin by using an effect (wrinkle removal, subcutaneous fat removal, skin elasticity improvement, and the like) occurring when high intensive acoustic energy is focused on a local site in a body by using high intensive focused ultrasound to increase temperature and thus a degenerated tissue is regenerated due to thermal variations occurring in the local site in the body.

Referring to FIG. 2, a skin treatment device 1 includes a main body 2 and a high intensive focused ultrasound probe 3 (hereinafter, referred to as an 'ultrasound probe' for convenience of explanation). The main body 2 controls the ultrasound probe 3. High intensive focused ultrasound is generated from the ultrasound probe 3 under the control of the main body 2, and the generated high intensive focused ultrasound can induce thermal variations by being focused on the inside of the body (for example, a dermis layer) through the ultrasound probe 3.

The main body 2 may include an input unit (not illustrated) for user input. The input unit may include not only a mouse and a keyboard but also a mechanical or electronic user interface (for example, a touch input-capable display) implemented in the device. Of course, the present disclosure is not limited thereto, and the input unit is applicable without particular limitations in the method and form thereof as long as the input unit is capable of inputting a user command.

The main body 2 may include an output unit (reference numeral is omitted) that displays information to the outside and transmits the information to a user. The output unit may include, for example, a display, an LED, a speaker, and the like for displaying visual output, auditory output, or tactile output. When the ultrasound probe 3 includes an imaging transducer element module, the output unit may display an ultrasound image of an internal tissue of the body.

The main body 2 may further include a peripheral device interface for data transmission with various types of external devices. For example, the main body 2 may include a memory card port, an external device input/output (I/O) port, and the like. The main body 2 can be connected to the ultrasound probe 3 through wired or wireless communication means to control the ultrasound probe 3.

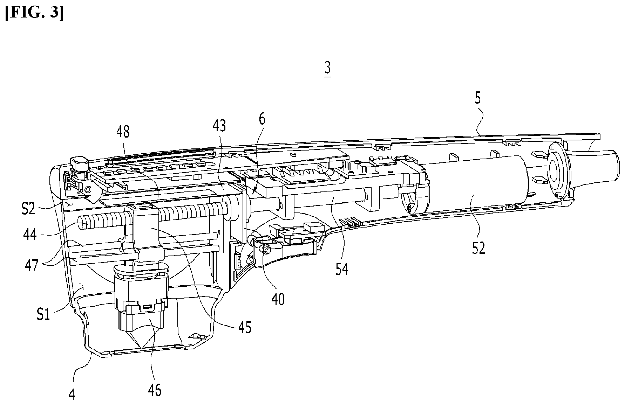

The ultrasound probe 3 may include a cartridge 4 and a handpiece 5. The cartridge 4 may be provided therein with a transducer 46 (see FIGS. 3 to 5) that generates high intensive focused ultrasound. The cartridge 4 provided with the transducer 46 can be detachably coupled to the handpiece 5 to be replaced with a cartridge 4 that generates high intensive focused ultrasound suitable for a treatment purpose or a medical procedure site and can be used.

The transducer 46 can generate high intensive focused ultrasound from an input power source and output the high intensive focused ultrasound. The cartridge 4 provided with the transducer 46 can be electrically connected or coupled to the handpiece 5 side, and the handpiece 5 can be connected to the main body by wire or wirelessly as described above, thereby exchanging signals or information (for example, transducer driving or control signals and transducer position information).

The cartridge 4 and the handpiece 5 can be electrically and physically connected to each other. The cartridge 4 and the handpiece 5 can be electrically connected to each other by connecting connection terminals (reference numerals are omitted) that are provided to correspond to each other when coupled, and can be physically coupled to each other through a predetermined coupling structure. The coupling structure may include, for example, a bar or a protrusion protruding in a direction in which the cartridge 4 is coupled to a front end of the handpiece 5.

With reference to FIGS. 3 to 5, the configuration of the high intensive focused ultrasound probe according to embodiments of the present disclosure is described.

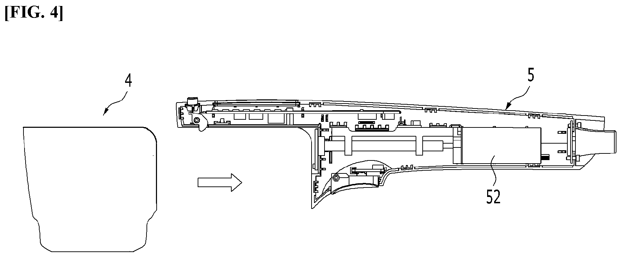

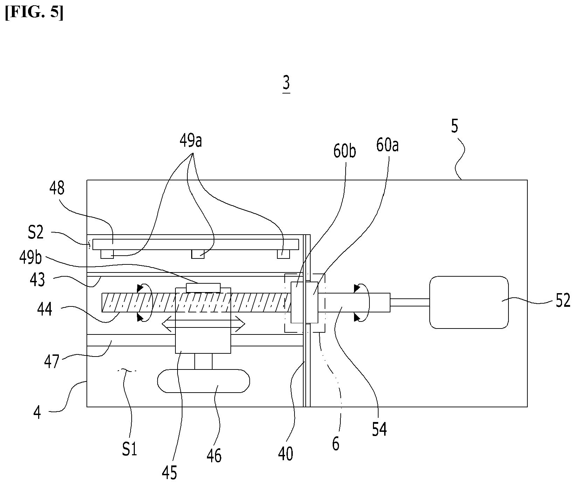

FIG. 3 is a cut perspective view illustrating an internal configuration of the high intensive focused ultrasound probe according to embodiments of the present disclosure and FIG. 4 is a view illustrating a state in which the cartridge is detached from the high intensive focused ultrasound probe illustrated in FIG. 3. FIG. 5 is a schematic view schematically illustrating a main configuration of the high intensive focused ultrasound probe according to embodiments of the present disclosure illustrated in FIG. 3.

Referring to FIGS. 3 to 5, the ultrasound probe 3 includes the cartridge 4 and the handpiece 5. The transducer 46 is provided inside the cartridge 4, and the cartridge 4 provided therein with the transducer 46 can be detachably coupled to the handpiece 5. Accordingly, the cartridge 4 can be replaced with a new one, or replaced with an appropriate cartridge 4 according to a treatment purpose or a medical procedure site.

The transducer 46 can generate high intensive focused ultrasound from an input power source and output the high intensive focused ultrasound. As described above, the cartridge 4 provided therein with the transducer 46 can be electrically connected or coupled to the handpiece 5 side. The handpiece 5 can be connected to the main body 2 (see FIG. 2) by wire or wirelessly, thereby exchanging signals or information (for example, transducer driving or control signals and transducer position information).

The cartridge 4 and the handpiece 5 can be electrically connected to each other by connecting electrical connection terminals (not illustrated) that are provided to correspond to each other when coupled. The cartridge 4 and the handpiece 5 can also be physically coupled to each other through a predetermined coupling structure. The coupling structure may include, for example, a bar or a protrusion (not illustrated) protruding in the direction in which the cartridge 4 is coupled to the front end of the handpiece 5.

According to an embodiment, the ultrasound probe 3 and the handpiece 5 may also be configured as an integrated type other than a detachable type as illustrated in the drawing (FIG. 4). In this case, the coupling structure (coupling structure such as a bar or a protrusion that protrudes in the direction in which the cartridge is coupled to the front end of the handpiece as a structure for physically coupling the cartridge and the handpiece) as in the detachable type may be deleted.

The inside of the cartridge 4 may be filled with a liquid ultrasound transmission medium (reference numeral is omitted). The liquid ultrasound transmission medium may be degassed water (water from which air bubbles have been removed). The transducer 46 can be arranged inside the cartridge 4 filled with the liquid ultrasound transmission medium. The transducer 46 can be provided to enable a one-dimensional motion, that is, a linear motion, inside the cartridge 4 in a specific direction (left and right direction based on FIG. 5).

A motor 52 can be installed in the handpiece 5 to implement a one-dimensional motion (linear motion) of the ultrasound transducer 46 in the specific direction. The transducer 46 can be mechanically connected to the motor 52 to receive power from the motor 52, and can generate high intensive focused ultrasound by making a linear reciprocating motion in the specific direction within the cartridge 4 by the received power, thereby focusing the generated high intensive focused ultrasound inside the body.

Referring to FIG. 5, a space partition plate 43 can be installed in the cartridge 4. An internal space of the cartridge 4 can be divided into a first space S1 and a second space S2 that are isolated from each other by the space partition plate 43. In the first space S1, the transducer 46 can be arranged and the liquid ultrasound transmission medium can be filled, and in the second space S2, a circuit board 48 that controls the operation of the transducer 46 from an input signal can be arranged.

In the first space S1, an internal rotating shaft 44 can be arranged together with the transducer 46. The internal rotating shaft 44 can be physically coupled to an external rotating shaft 54 arranged inside the handpiece 5 to receive rotational force from the external rotating shaft 54, and the transducer 46 can focus high intensive focused ultrasound inside the body while making a linear reciprocating motion in the first space S1 in the specific direction according to the rotation of the internal rotating shaft 44 by the external rotating shaft 54.

The external rotating shaft 54 can be directly connected to an output shaft (reference numeral is omitted) of the motor 52 inside the handpiece 5. The motor 52 can be arranged inside the handpiece 5 together with the external rotating shaft 54. The motor 52 can output rotational force in the forward/reverse direction according to a driving signal of the main body described above. Accordingly, the external rotating shaft 54 rotates in the forward or reverse direction within a set range, and as a result, the internal rotating shaft 44 can also be rotated in the same direction.

The internal rotating shaft 44 can be configured in the form of a lead screw having threads formed along its peripheral surface. The transducer 46 can be connected to the internal rotating shaft 44 in the first space S1 through a movable block 45, and the movable block 45 can be screw-coupled with the internal rotating shaft 44 through a fastening hole (hole formed with threads). Accordingly, the transducer 46 can make a linear motion in a specific direction together with the movable block 45 moving on the internal rotating shaft 44 when the internal rotating shaft 44 rotates.

A shaft 47 can be installed in the first space S1. The one-dimensional linear motion (linear motion according to the rotation of the internal rotating shaft 44) of the movable block 45 in the first space S1 can be stably implemented without shaking by the shaft 47. The shaft 47 can be arranged parallel to the internal rotating shaft 44 in the first space S1. The shaft 47 can be formed as one or more parallel to the internal rotating shaft 44.

A detection element 49a can be mounted on the circuit board 48 arranged in the second space S2. An element 49b to be detected can be provided on the movable block 45 arranged to be movable in the first space S1. The detection element 49a can recognize the element 49b to be detected and generate a corresponding signal to provide the generated signal to the circuit board 48, and a control circuit of the circuit board 48 can process the signal of the detection element 49a to calculate the value of a current position (position in the above specific direction) of the movable block 45.

The detection element 49a can be mounted on one surface of the circuit board 48, more specifically, on the surface of the circuit board 48 facing the movable block 45. At least two detection elements 49a can be mounted at a distance from each other on the one surface of the circuit board 48 along a direction parallel to the movement direction of the movable block 45, and the element 49b to be detected can be attached and fixed to the surface of the movable block 45 adj acent to the circuit board 48 or facing the circuit board 48.

The detection element 49a may be a Hall sensor. The element 49b to be detected may be a permanent magnet. In this case, the Hall sensor can detect the position of the permanent magnet on the movable block 45 by using the Hall effect and generate a corresponding signal to output the generated signal to the circuit board 48, and the circuit board 48 can recognize the position of the movable block 45 from the signal of the Hall sensor. The position value recognized in this way can be utilized to control the position of the movable block 45 by the motor 52.

The external rotating shaft 54 installed in the handpiece 5 and the internal rotating shaft 44 installed in the cartridge 4 can be physically coupled to be rotated in synchronization with each other. In embodiments of the present disclosure, the external rotating shaft 54 and the internal rotating shaft 44 can be indirectly coupled to each other through a magnet coupler 6 with a first sidewall 40 of the cartridge 4 interposed therebetween and can be rotated in synchronization with each other. More specifically, the external rotating shaft 54 and the internal rotating shaft 44 can be magnetically coupled to each other through the magnet coupler 6 with the first sidewall 40 interposed therebetween.

The magnetic coupler 6 may include a pair of couplers 60a and 60b. The pair of couplers may be a coupler installed on the external rotating shaft 54 (hereinafter, referred to as a 'first coupler 60a') and a coupler installed on the internal rotating shaft 44 (hereinafter, referred to as a 'second coupler 60b'). The first coupler 60a can be configured at an end of the external rotating shaft 54 adjacent to the first sidewall 40, and the second coupler 60b can be configured at an end of the internal rotating shaft 44 adjacent to the first sidewall 40.

The first coupler 60a can be configured as an integral portion or coupled portion at the end of the external rotating shaft 54, thereby enabling a rotational motion integrated with the external rotating shaft 54. The second coupler 60b can be configured as an integral portion or coupled portion at the end of the internal rotating shaft 44 and magnetically coupled with the first coupler 60a with the first sidewall 40 interposed therebetween, thereby enabling a rotational motion synchronized with the first coupler 60a.

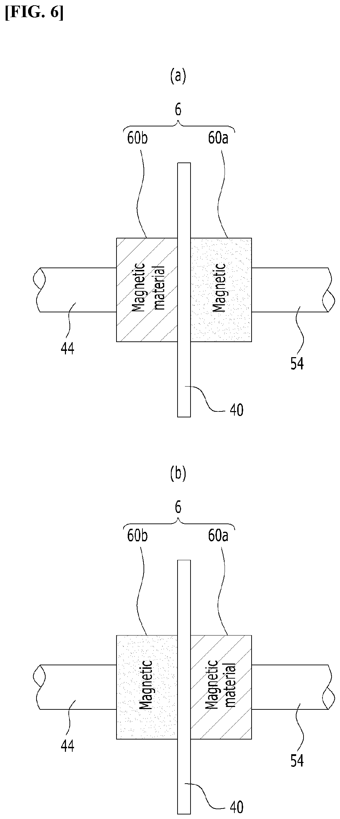

As an embodiment, for the magnetic coupling, one of the first coupler 60a and the second coupler 60b may be configured as a circular permanent magnet as illustrated in FIG. 6, and the other may be a circular magnetic material, for example, a steel plate. For example, when the first coupler 60a is a permanent magnet, the second coupler 60b may be a magnetic material (see FIG. 6A). In contrast, when the first coupler 60a is a magnetic material, the second coupler 60b may be a permanent magnet (see FIG. 6B).

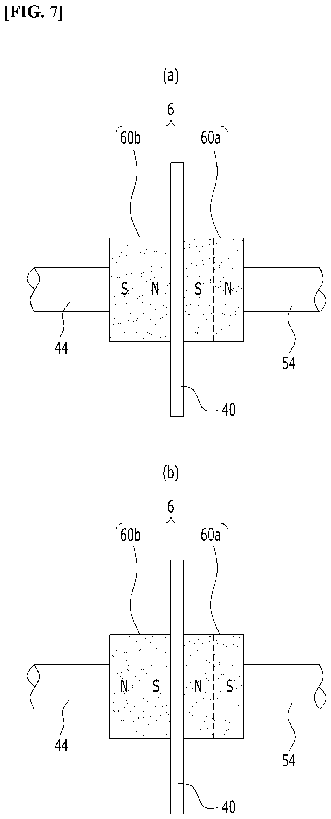

As another embodiment, the magnetic coupling can also be implemented by configuring both the first coupler 60a and the second coupler 60b as permanent magnets, preferably, circular permanent magnets, as illustrated in FIG. 7. In this case, the magnetic poles of the surface of the first coupler 60a and the surface of the second coupler 60b, which are permanent magnets that are in close contact with each other with the first sidewall 40 interposed therebetween, may be opposite to each other so that a mutually attractive force acts.

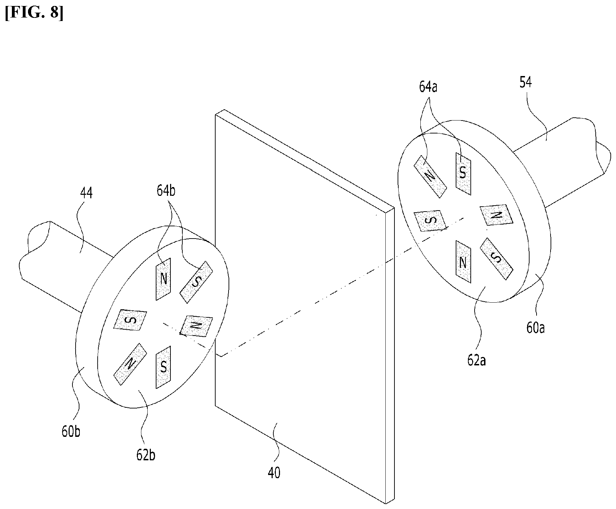

As further another embodiment, the magnetic coupling can also be implemented through a configuration in which coupling magnets 64a and 64b are separately mounted on a surface 62a of the first coupler 60a and a surface 62b of the second coupler 60b, the surface 62a and the surface 62b facing each other with the first sidewall 40 interposed therebetween, as illustrated in FIG. 8.

In this case, the coupling magnets 64a and 64b can be arranged at uniform intervals along the rotational direction on the surfaces 62a and 62b of the couplers 60a and 60b so that a relative slip in the rotational direction and a rotational force transmission delay due to the relative slip do not occur when a rotational force is transmitted from the first coupler 60a to the second coupler 60b.

The coupling magnets 64a mounted on the surface 62a of the first coupler 60a can be arranged so that the magnetic poles of sides exposed to the outside (sides in contact with the first sidewall) are alternated with respect to the rotation direction. The coupling magnets 64b mounted on the surface 62b of the second coupler 60b can also be arranged so that the magnetic poles of sides exposed to the outside (sides in contact with the first sidewall) are alternated with respect to the rotation direction.

The first coupler 60a and the second coupler 60b, which implement coupling between the shafts (the internal rotating shaft and the external rotating shaft), are indirectly connected through magnetic coupling with the first sidewall 40 interposed therebetween. Therefore, a portion of the first sidewall 40 that directly contacts each coupler may be worn during rotation. The wear is accelerated as the rotation speed increases, so that the life of the device may be shortened. Accordingly, it is necessary to reflect a method for suppressing or slowing down the wear in the configuration.

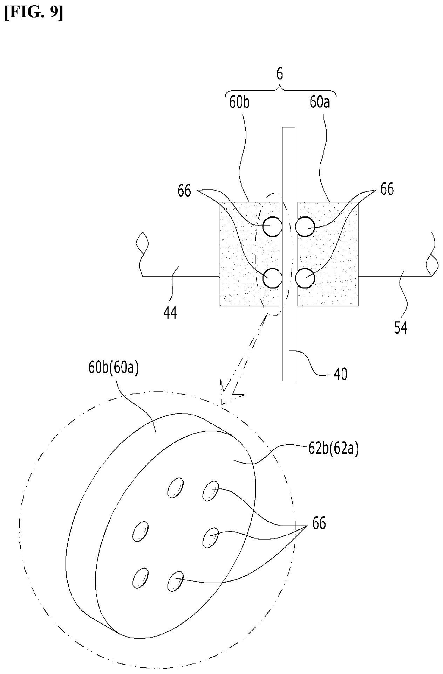

As an embodiment, as illustrated in FIG. 9, a plurality of balls 66 or needle pins may be installed on the surface of the first coupler 60a and the surface of the second coupler 60b facing each other with the first sidewall 40 interposed therebetween. At least a part of the balls 66 or the needle pins may protrude from the surfaces 62a and 62b facing each other. In this case, the protruding balls 66 or the needle pins support the rotational motion of the coupler while making point or line contact with the first sidewall 40 and performing a rolling motion, so that the wear of the first sidewall 40 can be significantly reduced.

Although not illustrated, as further another embodiment, the first and second couplers can be provided in the form of bearings. Inner rings of the first and second couplers mounted with the coupling magnets are formed with a narrower width than outer rings, so that even though the outer rings are fixed to the first sidewall, the inner rings can be spaced apart from the first sidewall by a predetermined distance. That is, even when the first and second couplers are rotated, no physical contact occur between the inner rings, so that the wear of the first sidewall can be avoided.

As described above, the first coupler 60a and the second coupler 60b are indirectly coupled by magnetic coupling with the first sidewall 40 interposed therebetween. Accordingly, the coaxiality between the two couplers may be distorted during the process of transmitting the rotational force, resulting in a loss of the rotational force. Therefore, it is necessary to reflect a method for stably maintaining the coaxiality between the two couplers in the configuration.

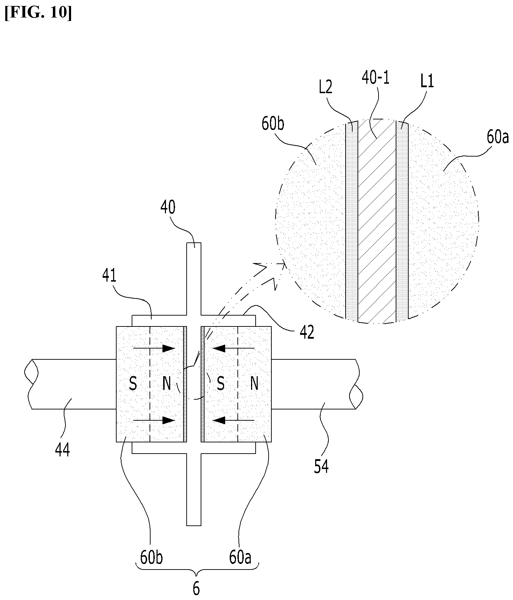

As an embodiment, as illustrated in FIG. 10, a ring-shaped internal rotation guide 41 can be formed on the inner surface of the first sidewall 40, and a ring-shaped external rotation guide 42 may be formed on the outer surface of the first sidewall 40 corresponding to the internal rotation guide 41. In this case, the second coupler 60b and the first coupler 60a can be respectively arranged in internal and external coupler receiving portions, which are respectively partitioned in the inside and the outside of the first sidewall 40 by the internal rotation guide 41 and the external rotation guide 42, thereby preventing coaxial misalignment.

In the embodiment of FIG. 10, a first lubricating layer L1 can be formed by a lubricant between the first coupler 60a and a first sidewall 40-1 partitioning the external coupler receiving portion. In addition, a second lubricating layer L2 can be formed by a lubricant between the second coupler 60b and the first sidewall 40-1 partitioning the inner coupler receiving portion. The lubricant is preferably a viscous semi-solid grease, but is not limited thereto.

According to such a configuration, direct contact between the pair of couplers 60a and 60b and the first sidewall 40-1 is blocked by the first lubricating layer L1 and the second lubricating layer L2, and thus the wear of the first sidewall 40 can be suppressed by the lubricating action of the lubricating layers. In addition, since the rotational load during the rotation of the couplers is reduced due to the lubricating action of the lubricating layers L1 and L2 and power consumption is reduced, thereby exhibiting an effect of improving the overall energy efficiency of the device.

In accordance with a high intensive focused ultrasound probe according to embodiments of the present disclosure as described above, the first coupler and the second coupler, which implement coupling between the shafts (the internal rotating shaft and the external rotating shaft), are not directly connected, but are indirectly connected through magnetic coupling with a sidewall (first sidewall) interposed therebetween. Accordingly, there is no need to process a hole (hole through which the shaft passes) in the cartridge filled with a liquid ultrasound transmission medium (for example, degassed water).

In addition, since the hole (hole through which the shaft passes) is not required, there is no need to consider a configuration (for example, the corrugated pipe-structured sealing member in the related art) for preventing leakage of the ultrasound transmission medium. That is, by adopting a power transmission method (magnetic coupling) that is structurally simple and requires no separate configuration for preventing the leakage of the ultrasound transmission medium (for example, degassed water) filled in the cartridge, there is an advantageous effect in improving the assembling efficiency and mass productivity of a product.

In addition, since the hole (hole through which the shaft passes) is not required in the cartridge, there is no need to consider sealing during a product design process, which can have the effect of improving the degree of freedom of design, and since an element (sealing member) for restricting a movable range is not required, the movable range of the transducer is increased compared to the configuration in the related art, which has the advantage of allowing ultrasound treatment or handling over a wider range with one-time procedure.

The above description is merely intended to illustratively describe the technical spirit of the present disclosure, and various changes and modifications can be made by those skilled in the art to which the present disclosure pertains without departing from the essential features of the present disclosure.

Therefore, the embodiments disclosed in the present disclosure are not intended to limit the technical spirit of the present disclosure, but are intended to describe the present disclosure. The scope of the technical spirit of the present disclosure is not limited by these embodiments. The scope of the present disclosure should be interpreted by the accompanying claims and all technical spirits falling within the equivalent scope thereto should be interpreted as being included in the scope of the present disclosure.

## Claims

1. A high intensive focused ultrasound probe including a cartridge that emits high intensity focused ultrasound to skin and a handpiece to which the cartridge is coupled, comprising:
an external rotating shaft configured to rotate inside the handpiece by a motor;
an internal rotating shaft configured to move a transducer while rotating inside the cartridge; and
a magnet coupler configured to magnetically couple the external rotating shaft and the internal rotating shaft with a first sidewall of the cartridge interposed between the external rotating shaft and the internal rotating shaft.

2. The high intensive focused ultrasound probe of claim 1, wherein the magnet coupler comprises:
a first coupler coupled to the external rotating shaft and making a synchronized rotational motion; and
a second coupler coupled to the internal rotating shaft and forming magnetic coupling with the first coupler with the first sidewall interposed between the first coupler and the second coupler.

3. The high intensive focused ultrasound probe of claim 2, wherein one of the first coupler and the second coupler is a magnetic material and the other one of the first coupler and the second coupler is a permanent magnet.

4. The high intensive focused ultrasound probe of claim 2, wherein the first coupler and the second coupler are permanent magnets, and a magnetic pole of a surface of the first coupler and a magnetic pole of a surface of the second coupler are opposite to each other, the two surfaces being in close contact with each other with the first sidewall interposed therebetween.

5. The high intensive focused ultrasound probe of claim 2, wherein a plurality of coupling magnets are separately mounted at uniform intervals along a rotation direction on a surface of the first coupler and a surface of the second coupler, the two surfaces facing each other with the first sidewall interposed therebetween.

6. The high intensive focused ultrasound probe of claim 5, wherein coupling magnets mounted on the surface of the first coupler are arranged so that magnetic poles of sides exposed to an outside are alternated with respect to the rotation direction, and
coupling magnets mounted on the surface of the second coupler are arranged so that magnetic poles of sides exposed to the outside are alternated with respect to the rotation direction.

7. The high intensive focused ultrasound probe of claim 2, wherein a plurality of balls or needle pins are installed on the surface of the first coupler and the surface of the second coupler, the two surfaces facing each other with the first sidewall interposed therebetween, and
at least a part of the balls or the needle pins protrude from the surfaces facing each other to support rotational motions of the first coupler and the second coupler in a state of being in contact with the first sidewall.

8. The high intensive focused ultrasound probe of claim 1, wherein a ring-shaped internal rotation guide is formed on an inner surface of the first sidewall where the magnet coupler is located,
a ring-shaped external rotation guide is formed on an outer surface of the first sidewall corresponding to the internal rotation guide, and
the magnet coupler is arranged in an internal coupler receiving portion and an external coupler receiving portion respectively partitioned in an inside and an outside of the first sidewall by the internal rotation guide and the external rotation guide.

9. The high intensive focused ultrasound probe of claim 8, wherein the magnet coupler comprises:
a first coupler coupled to the external rotating shaft and making a synchronized rotational motion; and
a second coupler coupled to the internal rotating shaft and forming magnetic coupling with the first coupler with the first sidewall interposed between the first coupler and the second coupler.

10. The high intensive focused ultrasound probe of claim 9, wherein a first lubricating layer is formed by a lubricant between the first coupler and a first sidewall of the external coupler receiving portion, and
a second lubricating layer is formed by a lubricant between the second coupler and a first sidewall of the internal coupler receiving portion.

11. The high intensive focused ultrasound probe of claim 1, wherein threads are formed on a peripheral surface of the internal rotating shaft,
a movable block having a fastening hole screw-coupled with the threads is coupled with the internal rotating shaft, and
the transducer is connected to the movable block.

12. The high intensive focused ultrasound probe of claim 11, wherein an internal space of the cartridge is divided by a space partition plate into a first space and a second space isolated from the first space.

13. The high intensive focused ultrasound probe of claim 12, wherein the first space is filled with a liquid ultrasound transmission medium,
the transducer and the internal rotating shaft are arranged in the first space filled with the liquid ultrasound transmission medium, and
a circuit board that controls the transducer is arranged in the second space.

14. The high intensive focused ultrasound probe of claim 13, wherein at least two detection elements that detect a position of the movable block are mounted at a distance from each other on the circuit board, and
an element to be detected is arranged on a surface of the movable block adjacent to the circuit board.

15. The high intensive focused ultrasound probe of claim 14, wherein the detection element is a Hall element, and
the element to be detected is a permanent magnet.

16. The high intensive focused ultrasound probe of claim 12, wherein in the first space, a shaft that guides a one-dimensional linear motion of the movable block is arranged.
